(19)

Europäisches Patentamt
European Patent Office
Office européen des brevets

(11) Veröffentlichungsnummer: **0 348 341 B1**

(12) **EUROPÄISCHE PATENTSCHRIFT**

(45) Veröffentlichungstag der Patentschrift: **23.11.94**

(51) Int. Cl.5: **C07D 209/12**, A61K 31/40

(21) Anmeldenummer: **89730151.1**

(22) Anmeldetag: **23.06.89**

(54) **Aminoalkylindole, Verfahren zu deren Herstellung und diese enthaltende pharmazeutische Präparate.**

(30) Priorität: **23.06.88 DE 3821148**

(43) Veröffentlichungstag der Anmeldung:
**27.12.89 Patentblatt 89/52**

(45) Bekanntmachung des Hinweises auf die Patenterteilung:
**23.11.94 Patentblatt 94/47**

(84) Benannte Vertragsstaaten:
**AT BE CH DE ES FR GB GR IT LI LU NL SE**

(56) Entgegenhaltungen:
**EP-A- 0 262 498**
**FR-A- 2 512 446**

(73) Patentinhaber: **SCHERING AKTIENGESELLSCHAFT**

**D-13342 Berlin (DE)**

(72) Erfinder: **von Angerer, Erwin, Dr.**
**Regensburger Strasse 6**
**D-8401 Grasslfing (DE)**

**EP 0 348 341 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft Aminoalkylindole, Verfahren zu deren Herstellung und diese enthaltende pharmazeutische Präparate.

Die neuen Aminoalkylindole werden durch die allgemeine Formel I gekennzeichnet

(I) ,

worin

- $R_1$ ein Wasserstoffatom, eine Hydroxyl- oder eine Alkanoyloxygruppe mit 1-10 Kohlenstoffatomen,
- $R_2$ eine Hydroxyl- oder Alkanoyloxygruppe mit 1-10 Kohlenstoffatomen,
- $R_3$ ein Wasserstoffatom oder eine Methylgruppe,
- $R_4$, $R_5$ Wasserstoff, Alkylgruppen mit 1-10 Kohlenstoffatomen, Aralkylgruppen mit 7-10 Kohlenstoffatomen oder Cycloalkylgruppen mit 3-7 Kohlenstoffatomen, wobei die Reste $R_4$ und $R_5$ gleich oder verschieden sind oder
- $R_4$, $R_5$ unter Einschluß des N-Atoms einen 5- oder 6-gliedrigen heterocyclischen Ring bilden, der gegebenenfalls neben dem Stickstoffatom noch ein Sauerstoff- oder ein weiteres Stickstoffatom als Heteroatom enthält und
- $n = 4\text{-}15$

bedeuten, und die Salze dieser Verbindungen mit Säuren.

Als Alkanoyloxygruppen $R_1$ und $R_2$ kommen Reste von organischen Carbonsäuren mit 1-10 Kohlenstoffatomen infrage, die gesättigt oder ungesättigt sein können. Sie leiten sich ab von aliphatischen, cycloaliphatischen, aliphatisch-cycloaliphatischen, cycloaliphatisch-aliphatischen und aromatischen Monocarbonsäuren. Die Anzahl der Kohlenstoffatome im Ring variiert von 3 bis 7. Bevorzugt werden als Reste $R_1$ und $R_2$ die Alkanoyloxygruppen der Essig-, Propion-, Butter-, Isobutter-, Pivalin-, Capron-, Acryl-, Croton-, Heptyl-, Capryl-, Pelargon-, Decan-, 3-Cyclopentylpropion- und Benzoesäure.

Der Rest $R_1$ kann sich in den Positionen 4, 5, 6 und 7 des Indolringsystems befinden, besonders geeignet ist die 5-Position.

Der Rest $R_2$ kann sich in der Position 2, 3 und 4 am Phenylring, wenn die Position 1 die Verknüpfungsstelle zur 2-Position des Indolsystems ist, befinden. Es sind als Wirkstoffe solche Verbindungen besonders geeignet, bei denen der Rest $R_2$ in der 4-Position (para-Stellung) steht.

Alkylgruppen mit 1-10 Kohlenstoffatomen und Cycloalkylgruppen mit 3-7 Kohlenstoffatomen kommen als Reste $R_4$ und $R_5$ infrage.

Als Alkylgruppen sind die Reste Methyl, Ethyl, Propyl, Butyl, Pentyl, Hexyl, Heptyl, Octyl, Nonyl und Decanyl geeignet. Als Cycloalkylgruppen sind besonders die Reste Cyclopentyl und Cyclohexyl zu nennen.

Als Beispiel einer Aralkylgruppe für die Reste $R_4$ und/oder $R_5$ ist insbesondere die Benzylgruppe zu nennen.

Die Reste $R_4$ und $R_5$ können gleich oder verschieden oder Bestandteile eines gemeinsamen Ringes sein. Beim Vorliegen eines Ringes kann dieser neben dem Stickstoffatom zusätzlich ein Sauerstoffatom und ein zweites Stickstoffatom enthalten. Als solche Reste sind besonders geeignet die Kombination Wasserstoff/Methyl (abgeleitet von Methylamino), Wasserstoff/Wasserstoff (abgeleitet von Amino), Methyl/Methyl (abgeleitet von Dimethylamino), sowie der $-(CH_2)_4$-Rest (abgeleitet von Pyrrolidino), der $-(CH_2)_5$-Rest (abgeleitet von Piperidino), der $-(CH_2)_2-O-(CH_2)_2$-Rest (abgeleitet von Morpholino) und der $-(CH_2)_2-NH-(CH_2)_2$-Rest (abgeleitet von Piperazino).

Die Erfindung betrifft auch Salze der Verbindungen der allgemeinen Formel I. Diese Salze können sowohl Salze von anorganischen als auch von organischen Säuren sein. Als besonders geeignet sind die physiologisch gut verträglichen Salze, wie beispielsweise Hydrochloride, Hydrobromide, Acetate, Malonate

und Citrate.

Die Erfindung betrifft ferner Verfahren zur Herstellung von Aminoalkylindolen der allgemeinen Formel I. Dabei wird eine Verbindung der allgemeinen Formel II a

(II a) ,

oder der allgemeinen Formel II b

(II b),

worin

| | |
|---|---|
| $R_3$, $R_4$, $R_5$ und n | die in Formel I genannte Bedeutung haben, |
| $R_6$ | ein Wasserstoffatom oder eine Alkoxygruppe mit 1-4 Kohlenstoffatomen und |
| $R_7$ | eine Alkoxygruppe mit 1-4 Kohlenstoffatomen |

bedeuten,

unter den Bedingungen einer Etherspaltung zu einer Verbindung der Formel I a

(I a) ,

oder der Formel III b

(III b) ,

worin $R_8$ ein Wasserstoffatom oder eine Hydroxylgruppe bedeuten,
umsetzt, die Carbonylfunktion der Verbindung der allgemeinen Formel III b vollständig reduziert und anschließend gegebenenfalls die freie(n) aromatische(n) Hydroxylgruppe(n) in eine Alkanoyloxygruppe mit 1-10 Kohlenstoffatomen und gegebenenfalls eine Verbindungen der allgemeinen Formel I mit einer organischen oder anorganischen Säure in das entsprechende Salz überführt.

Als Alkoxygruppen der Reste $R_6$ und $R_7$ kommen Solche mit 1-4 Kohlenstoffatomen infrage, wie beispielsweise die Methoxy-, Ethoxy-, Propoxy-, Isopropoxy-, Butoxy-, Isobutoxy- und tert. -Butoxygruppe.

Solche Verbindungen der Formel II, worin $R_6$ und $R_7$ Alkoxygruppen mit 1-4 Kohlenstoffatomen bedeuten, können durch Etherspaltung in die entsprechenden Hydroxyverbindungen der allgemeinen Formel I überführt werden. Die Etherspaltung erfolgt mit und ohne Lösungsmittel. Als Reagenzien für die Etherspaltung eignen sich Bortribromid, Bortrifluorid, Aluminiumtrichlorid, Siliziumtetrachlorid, Aluminiumtribromid, Natriummethylthiolat und Trimethylsilyljodid. Die Reaktion wird bei Temperaturen zwischen -70 °C und 200 °C durchgeführt. Als Lösungsmittel für diese Etherspaltung kommen inerte Lösungsmittel infrage. Zu nennen sind aliphatische Halogenkohlenwasserstoffe, wie zum Beispiel Methylenchlorid, aromatische Kohlenwasserstoffe, wie Benzol, Toluol, Xylol, halogenierte aromatische Kohlenwasserstoffe, wie Chlorbenzol, Dichlorbenzole und Dimethylformamid sowie Acetonitril. Es eignen sich aber auch aliphatische Ether mit Alkylresten aus 1-6 Kohlenstoffatomen.

Weiterhin kann diese Etherspaltung auch mittels konzentrierter Jod-Wasserstoffsäure, Pyridinhydrochlorid, Bromwasserstoffsäure und Methylmagnesiumjodid bei Temperaturen zwischen 20 °C bis 250 °C erfolgen.

Für die sich gegebenenfalls anschließende Veresterung der phenolischen Hydroxylgruppen kommen die üblicherweise in der Chemie zur Veresterung angewendeten Verfahren infrage. Beispielsweise sei die Umsetzung mit einer Carbonsäure oder eines Carbonsäureanhydrides in Gegenwart starker Säuren, wie zum Beispiel Trifluoressigsäure, Perchlorsäure oder p-Toluolsulfonsäure, bei Raumtemperatur oder etwas angehobener Temperatur oder die Umsetzung mit einem Carbonsäureanhydrid in Gegenwart eines tertiären Amins bei etwa 20-80 °C zu nennen.

Werden Pyridin und 4-Dimethylamino-pyridin als tertiäre Amine gemeinsam angewandt, kann die Veresterung vorzugsweise bei Raumtemperatur durchgeführt werden.

Die sich gegebenenfalls anschließende Salzbildungsreaktion erfolgt nach den üblichen Standardvorschriften.

Es wurde gefunden, daß sowohl die erfindungsgemäßen Verbindungen gemäß allgemeiner Formel I als auch die erfindungsgemäßen Verbindungen der allgemeinen Formel III b stark antiöstrogene Eigenschaften besitzen. Daher betrifft die Erfindung auch die Verbindungen der vorstehend genannten allgemeinen Formel III b.

Verbindungen mit antiöstrogenen Eigenschaften, d.h. Stoffe mit Hemmwirkungen gegenüber Östrogenen, werden bereits in der Literatur beschrieben.

Als Antiöstrogen ist beispielsweise das Tamoxifen zu nennen (Eur.J. Cancer Clin. Oncol, 1985, 21, 985 und J.S. Patterson, "10 Years of Tamoxifen in Breast Cancer" in Hormonal Manipulation of Cancer; Peptides, Growth Factors and New (Anti) Steroidal Agents, Raven Press, New York (1987)).

Steroidale Antiöstrogene werden in der europäischen Patentanmeldung, Veröffentlichungsnummer 0138 504 beschrieben. Weitere Antiöstrogene, die wie die vorliegende Erfindung Indolderivate betreffen, werden bereits in der deutschen Patentschrift 32 32 968, im J.Med.Chem. 1983, 26, 113, J.Med.Chem., 1984, 27, 1439, Eur.J. Cancer. Clin. Oncol. 1985, 21, 531 und Cancer Treatment Reviews 1984, 11, 147 beschrieben. Hydroxylierte 2-Phenylindole, die in Form von Diamin-platin (II)-Komplex-Verbindungen vorliegen, werden in der deutschen Offenlegungsschrift 37 30 746 genannt.

Als Nachteil der bisher bekannten Antiöstrogene ist festzustellen, daß diese neben der antiöstrogenen Wirkkomponente auch eine mehr oder weniger stark ausgeprägte östrogene Wirkkomponente besitzen, die als unerwünscht angesehen wird.

Die Aufgabe der vorliegenden Erfindung liegt darin, Verbindungen bereitzustellen, die bedeutend reinere Antiöstrogene als die bereits bekannten Verbindungen darstellen, d.h. daß sie starke antiöstrogene Effekte bei nur minimaler östrogener Restwirkung aufweisen.

Diese gestellte Aufgabe wird durch die Bereitstellung der Verbindungen der allgemeinen Formel I gelöst; auch die erfindungsgemäßen (Zwischen-)Verbindungen der allgemeinen Formel III b genügen - in gewissen Grenzen - der vorstehend genannten Forderung.

Die erfindungsgemäßen Verbindungen der allgemeinen Formeln I besitzen eine ausgeprägte Affinität zum Östradiol-Rezeptor und verdrängen kompetiv $^3$H-17$\beta$-Östradiol vom Rezeptor.
In vivo besitzen sie starke antiöstrogene Efekte am Uterus der Maus und hemmen das östron-stimulierte Uteruswachstum bis zu 100 %. Östrogene Wirkungen sind in diesen Tests nicht oder nur in sehr geringem

Maße nachweisbar. Die Verbindungen wirken hemmend auf das Wachstum von hormonabhängigen Tumorzellen, insbesondere hemmen sie das Wachstum von östrogen-abhängigen menschlichen Mammatumorzellen (MCF-7).

Die erfindungsgemäßen Verbindungen eignen sich zur Therapie von östrogen-abhängigen Erkrankungen, zum Beispiel anovulatorischer Infertilität, Prostatahyperplasie, Mammacarcinom, Endometriumcarcinom und Melanom.

Die folgenden pharmakologischen Tests zeigen die Wirksamkeit der erfindungsgemäßen Verbindungen.

Tabelle 1 zeigt eine Übersicht der getesteten Verbindungen der allgemeinen Formeln I und deren relativen Bindungsaffinitäten (RBA*) zum Östrogenrezeptor aus Kalbsuteri, bezogen auf 17$\beta$-Östradiol = 100.

Die Testanordnung wird in Cancer Treatment Reviews 1984, 11, 147 beschrieben.

Aus der Tabelle 1 geht hervor, daß die Verbindungen 2, 3, 6 und 8 die größten Affinitäten im Vergleich zu Östradiol zeigen. Die Verbindung 7 zeigte in dieser getesteten Reihe die geringste Affinität zum Östrogenrezeptor.

Tabelle 1

Getestete Verbindungen der Formel I, und deren relative Bindungsaffinitäten zum Östrogenrezeptor

| Verbindung | n | $R_3$ | $R_4$ | $R_5$ | RBA* |
|---|---|---|---|---|---|
| 1 | 4 | $CH_3$ | $-(CH_2)_4-$ | | 3.6 |
| 2 | 6 | $CH_3$ | H | H | 25 |
| 3 | 6 | $CH_3$ | $CH_3$ | H | 27 |
| 4 | 6 | $CH_3$ | $CH_3$ | $CH_3$ | 12 |
| 5 | 6 | H | $-(CH_2)_4-$ | | 2.3 |
| 6 | 6 | $CH_3$ | $-(CH_2)_4-$ | | 21 |
| 7 | 6 | H | $-(CH_2)_5-$ | | 1.3 |
| 8 | 6 | $CH_3$ | $-(CH_2)_5-$ | | 23 |
| 9 | 8 | $CH_3$ | $-(CH_2)_4-$ | | 7.6 |
| 10 | 6 | $CH_3$ | $-(CH_2)_2-O-CH_2)_2-$ | | 10 |
| 11 | 6 | $CH_3$ | $C_2H_5$ | $C_2H_5$ | 14 |
| 12 | 6 | $CH_3$ | $CH_2C_6H_5$ | H | 15 |

* Relative Bindungsaffinitäten zum Östrogenzeptor aus Kalbsuteri, bezogen auf 17ß-Östradiol = 100

Tabelle 2 zeigt die ostrogenen und antiöstrogenen Wirksamkeiten der Verbindungen 1, 2, 3, und 6. Diese Wirksamkeiten wurden in einem in-vivo-Test an der infantilen Maus gefunden. Dieser Test wird ausführlich in Cancer Treatment Reviews 1984, 11, 147 und J.Med.Chem., 1984, 27, 1439 beschrieben. Aus der Tabelle geht hervor, daß die Verbindungen 1, 2, 3 und 6 einen starken bis sehr starken östrogenantagonistischen Effekt zeigen. Bei allen getesteten Derivaten, mit Ausnahme der Verbindung 3, ist eine östrogene Eigenwirkung kaum nachweisbar.

**Tabelle 2**

**Uterotrophe und antiuterotrophe Wirkung an der infantilen Maus**

| | | Uterotropher Test | Antiuterotropher Test | |
|---|---|---|---|---|
| Verb. | Dosis (µg) | Rel. Uterusgew. | Rel. Uterusgew. | Hemmung (%) |
| Kontrolle | | 7.7 ± 2.5 | | |
| 1 | 1 | 7.6 ± 2.8 | 43.7 ± 8.2 | 5 |
| | 5 | 8.0 ± 3.1 | 47.6 ± 10.5 | |
| | 25 | 6.4 ± 1.1 | 16.2 ± 4.6 | 78 |
| | 125 | 6.4 ± 1.3 | 25.5 ± 7.9 | 53 |
| Östron | 0.4 | 45.8 ± 6.2 | | |
| Kontrolle | | 15.9 ± 3.6 | | |
| 2 | 1 | 10.9 ± 4.7 | | |
| | 5 | 12.0 ± 2.2 | 30.0 ± 9.1 | 68 |
| | 25 | 12.4 ± 2.7 | 15.1 ± 1.2 | 102 |
| | 125 | 16.9 ± 2.7 | 16.2 ± 2.7 | 99 |
| 3 | 1 | 14.2 ± 3.1 | | |
| | 5 | 22.8 ± 8.6 | 38.1 ± 8.2 | 49 |
| | 25 | | 41.0 ± 8.5 | 43 |
| | 125 | 34.0 ± 6.3 | 33.8 ± 2.4 | 59 |
| Östron | 0.4 | 59.8 ± 20.0 | | |
| Kontrolle | | 15.5 ± 2.9 | | |
| 6 | 1 | 12.5 ± 1.7 | 45.8 ± 3.2 | 20 |
| | 5 | 9.0 ± 1.8 | 15.4 ± 1.7 | 100 |
| | 25 | 13.9 ± 2.8 | 14.4 ± 2.2 | 103 |
| | 125 | | 8.9 ± 1.6 | 117 |
| Östron | 0.4 | 53.4 ± 3.6 | | |
| Kontrolle | | 11.5 ± 3.6 | | |
| 6 | 1 | | 48.3 ± 7.2 | |
| | 5 | | 20.1 ± 7.3 | 73 |
| | 25 | | 12.6 ± 1.9 | 97 |
| | 50 | 8.2 ± 1.8 | | |
| | 250 | 12.7 ± 2.7 | | |
| Östron | 0.4 | 43.0 ± 7.2 | | |

Tabelle 2 (Fortsetzung)

Uterotrophe und antiuterotrophe Wirkung an der infantilen Maus

| Verb. | Dosis (µg) | Uterotropher Test Rel. Uterusgew. | Antiuterotropher | Rel. Uterusgew. | Test Hemmung (%) |
|---|---|---|---|---|---|
| Kontrolle | | 18.8±9.6 | | 18.8±9.6 | |
| 10 | 1 | 21.2±3.9 | | | |
| | 5 | 19.5± 6.9 | 5 | 51.2±7.5 | 8 |
| | 25 | 21.8±11.3 | 25 | 47.7±8.4 | 18 |
| | 125 | 17.4± 1.7 | 125 | 18.0±4.8 | 102 |
| Östron | 0.4 | 54.2±12.1 | 0.4 | 54.2±12.1 | |
| | Dosis: µg/Tier | Wirkung: | Dosis: µg/Tier | Wirkung: | Hemmung/%: |
| Kontrolle | | 25.4±14.5 | | 25.4±14.5 | |
| 11 | 5 | 24.8±11.7 | 5 | 51.3±4.9 | 11 |
| | 25 | 14.7± 4.3 | 25 | 52.1±8.9 | 8 |
| | 125 | 19.8± 3.3 | 125 | 40.0±4.7 | 50 |
| | 625 | 36.4±17.6 | 625 | 43.7±7.4 | 37 |
| Östron | 0.4 | 54.6±6.5 | 0.4 | 54.6±6.5 | |
| | Dosis: µg/Tier | Wirkung: | Dosis: µg/Tier | Wirkung: | Hemmung/%: |
| Kontrolle | | 18.2±5.5 | | 18.2±5.5 | |
| 12 | 5 | 17.8±4.6 | 5 | 53.2±9.0 | 25 |
| | 25 | 23.6±2.5 | 25 | 44.6±7.1 | 43 |
| | 125 | 34.0±7.2 | 125 | 40.8±8.7 | 51 |
| | | | 625 | 41.8±7.4 | 49 |
| Östron | 0.4 | 64.7±8.7 | 0.4 | 64.7±8.7 | |

Tabelle 3 zeigt die Ergebnisse der Untersuchungen zur cytostatischen Aktivität der Verbindungen 1 und 6 im Vergleich zu Tamoxifen.

An hormonsensitiven menschlichen MCF-7 Mammacarcinomzellen wurde eine starke Hemmung des Zellwachstums, bestimmt durch Zellzahl und [$^3$H]-Thymidineinbau, in die DNA gefunden. Bei Konzentrationen von $10^{-7}$M wurde eine signifikante Erniedrigung der Zellzahl und des Thymidineinbaus 6 Tage nach

Inkubation gefunden. Unter gleichen Bedingungen zeigte Tamoxifen keinen signifikanten Effekt.

Die zur Untersuchung verwendete Testanordnung wurde in Eur.J.Cancer Clin. Oncol. 1985, 21, 531 ausführlich beschrieben.

Tabelle 3

| **Wirkung von 1 und 6 auf das Wachstum von hormonabhängigen menschlichen MCF-7 Mammacarcinomzellen** | | | |
|---|---|---|---|
| Verb. | Konz. (M) | Zellzahl (% T/C) | [$^3$H]Thymidineinbau (% T/C) |
| 1 | $1 \times 10^{-7}$ | 106 | 67 |
| | $5 \times 10^{-7}$ | 84 | 76 |
| | $1 \times 10^{-6}$ | 94 | 85 |
| | $5 \times 10^{-6}$ | 62 | 74 |
| 6 | $1 \times 10^{-7}$ | 65 | 32 |
| | $5 \times 10^{-7}$ | 49 | 32 |
| | $1 \times 10^{-6}$ | 24 | 22 |
| | $5 \times 10^{-6}$ | 12 | 3 |
| 8 | $1 \times 10^{-7}$ | 48 | |
| | $1 \times 10^{-6}$ | 44 | |
| | $1 \times 10^{-5}$ | 38 | |
| 10 | $1 \times 10^{-7}$ | 75 | |
| | $1 \times 10^{-6}$ | 52 | |
| | $1 \times 10^{-5}$ | 36 | |
| 12 | $1 \times 10^{-7}$ | 47 | |
| | $1 \times 10^{-6}$ | 46 | |
| | $1 \times 10^{-5}$ | 41 | |
| Tamoxifen | $1 \times 10^{-7}$ | 77 | 71 |
| | $1 \times 10^{-6}$ | 27 | 27 |

Die Tabellen 4, 5 und 6 zeigen in Analogie zu den Tabellen 1, 2, und 3 die entsprechenden Daten für die getesteten 1-Carbamoyl-alkyl-2-phenylindole der allgemeinen Formel III b.
Wie aus Tabelle 5 hervorgeht, weisen diese Verbindungen deutliche Antiöstrogenitat auf, besitzen allerdings in hoherer Dosierung auch eine signifikante östrogene Partialwirkung.

**Tabelle 4**

**Getestete 1-Carbamoylalkyl-2-phenylindole der allgemeinen Formel III b und deren relative Bindungsaffinitäten zum Östrogenrezeptor**

| Verbindung | $R_4$ | $R_5$ | RBA |
|---|---|---|---|
| 16 | $-(CH_2)_4-$ | | 7 |
| 17 | $-(CH_2)_5-$ | | 19 |
| 18 | $-(CH_2)_2-O-(CH_2)_2-$ | | 26 |
| 19 | $C_2H_5$ | $C_2H_5$ | 15 |
| 20 | $CH_2C_6H_5$ | H | 12 |

**Tabelle 5**

**Uterotrophe und antiuterotrophe Wirkung der 1-Carbamoylalkyl-2-phenylindole** der allgemeinen Formel III b an der infantilen Maus

| Verbindung | Uterotropher Test | | Antiuterotropher Test | | |
|---|---|---|---|---|---|
| | Dosis: µg/Tier | Wirkung: | Dosis: µg/Tier | Wirkung: | Hemmung/%: |
| Kontrolle | | 21.9±2.7 | | 21.9±2.7 | |
| 16 | 5 | 18.2± 2.3 | 5 | 47.5±10.9 | 10 |
| | 25 | 16.5± 2.4 | 25 | 42.0± 7.0 | 29 |
| | 125 | 17.7± 0.9 | 125 | 35.2± 5.5 | 53 |
| | 625 | 35.3±10.7 | 625 | 40.7± 3.9 | 44 |
| Östron | 0.4 | 50.2±3.1 | 0.4 | 50.2±3.1 | |

| Verbindung | Dosis: µg/Tier | Wirkung: | Dosis: µg/Tier | Wirkung: | Hemmung/%: |
|---|---|---|---|---|---|
| Kontrolle | | 14.4±2.5 | | 14.4±2.5 | |
| 17 | 1 | 23.8±2.8 | | | |
| | 5 | 17.7±3.2 | 5 | 48.7±9.1 | 6 |
| | 25 | 16.3±3.0 | 25 | 42.9±4.7 | 22 |
| | 125 | 18.6±2.5 | 125 | 29.5±2.7 | 59 |
| Östron | 0.4 | 50.8±7.3 | 0.4 | 50.8±7.3 | |

Fortsetzung Tabelle 5:

| Verbindung | Uterotropher Test Dosis: µg/Tier | Wirkung: | Antiuterotropher Test Dosis: µg/Tier | Wirkung: | Hemmung/%: |
|---|---|---|---|---|---|
| Kontrolle | | 18.8±9.6 | | 18.8±9.6 | |
| 18 | 1 | 20.2±3.4 | | | |
| | 5 | 22.0±5.6 | 5 | 53.5± 8.7 | |
| | 25 | 21.2±4.6 | 25 | 52.8± 7.4 | |
| | 125 | 43.0±8.2 | 125 | 39.8±18.1 | 41 |
| Östron | 0.4 | 54.2±12.1 | 0.4 | 54.2±12.1 | |

| | Dosis: µg/Tier | Wirkung: | Dosis: µg/Tier | Wirkung: | Hemmung/%: |
|---|---|---|---|---|---|
| Kontrolle | | 25.4±14.5 | | 25.4±14.5 | |
| 19 | 5 | 18.4±3.2 | 5 | 50.9±8.2 | 13 |
| | 25 | 17.9±2.0 | 25 | 50.9±4.8 | 13 |
| | 125 | 17.6±2.6 | 125 | 44.5±6.2 | 34 |
| | 625 | 32.7±6.5 | 625 | 35.3±6.1 | 66 |
| Östron | 0.4 | 54.6±6.5 | 0.4 | 54.6±6.5 | |

| | Dosis: µg/Tier | Wirkung: | Dosis: µg/Tier | Wirkung: | Hemmung/%: |
|---|---|---|---|---|---|
| Kontrolle | | 15.6±5.8 | | 15.6±5.8 | |
| 20 | 5 | 16.5±3.6 | 5 | 50.7± 5.0 | |
| | 25 | 20.9±6.5 | 25 | 46.9± 7.7 | 12 |
| | 125 | 16.7±3.9 | 125 | 50.3± 4.6 | |
| | 625 | 19.9±4.1 | 625 | 59.5±12.4 | -23 |
| Östron | 0.4 | 51.2±5.9 | 0.4 | 51.2±5.9 | |

Tabelle 6

| Wirkung der 1-Carbamoylalkyl-2-phenylindole der allgemeinen Formel III b auf das Wachstum von hormonabhängigen menschlichen MCF-7 Mammacarcinomzellen | | |
|---|---|---|
| Verbindung: | Konzentration (M): | Zellzahl (%T/C): |
| 16 | $1 \cdot 10^{-7}$ | 57.2±4.4 |
| | $5 \cdot 10^{-7}$ | 48.2±5.4 |
| | $1 \cdot 10^{-6}$ | 47.1±3.7 |
| | $5 \cdot 10^{-6}$ | 44.0±3.6 |
| | $1 \cdot 10^{-5}$ | 42.1±4.0 |
| 17 | $1 \cdot 10^{-7}$ | 89.0±10.8 |
| | $5 \cdot 10^{-7}$ | 95.5±10.2 |
| | $1 \cdot 10^{-6}$ | 96.0± 7.7 |
| | $5 \cdot 10^{-6}$ | 99.6± 7.6 |
| | $1 \cdot 10^{-5}$ | 86.5± 8.3 |
| 18 | $1 \cdot 10^{-7}$ | 57.5±6.8 |
| | $5 \cdot 10^{-7}$ | 58.1±6.7 |
| | $1 \cdot 10^{-6}$ | 56.4±5.2 |
| | $5 \cdot 10^{-6}$ | 42.4±3.7 |
| | $1 \cdot 10^{-5}$ | 33.3±2.8 |
| 19 | $1 \cdot 10^{-7}$ | 59.7±6.1 |
| | $5 \cdot 10^{-7}$ | 60.1±3.8 |
| | $1 \cdot 10^{-6}$ | 61.4±4.4 |
| | $5 \cdot 10^{-6}$ | 56.2±4.5 |
| | $1 \cdot 10^{-5}$ | 44.9±3.7 |
| 20 | $1 \cdot 10^{-7}$ | 58.3±4.9 |
| | $5 \cdot 10^{-7}$ | 47.9±3.0 |
| | $1 \cdot 10^{-6}$ | 46.8±4.2 |
| | $5 \cdot 10^{-6}$ | 36.6±2.5 |
| | $1 \cdot 10^{-5}$ | 26.8±4.2 |

Die Erfindung betrifft auch pharmazeutische Präparate, die mindestens eine Verbindung der allgemeinen Formel I oder III b, sowie deren Salze enthalten und die Verwendung dieser Verbindungen zur Behandlung von östrogenabhängigen Krankheiten und Tumoren.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zur verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01-100 mg/kg Körpergewicht, vorzugsweise 0,1-20 mg/kg Körpergewicht, je Tag betragen.

Zur oralen Verabreichung kommen Kapseln, Pillen, Tabletten, Dragées usw. infrage. Die Dosierungseinheiten können neben dem Wirkstoff einen pharmazeutisch verträglichen Träger, wie zum Beispiel Stärke,

Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl. Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

**Herstellung der Ausgangsmaterialien**

Die für die Herstellung der erfindungsgemäßen Verbindungen der allgemeinen Formel I zu verwendenden Ausgangsprodukte der allgemeinen Formel II a bzw. II b werden

A) entweder durch N-Alkylierung der entsprechenden 1H-2-Phenylindolverbindung X mit dem entsprechenden $\alpha,\omega$-Dibromalkan $Br\text{-}(CH_2)_n\text{-}Br$ und nachfolgendem Austausch des $\omega$-Br-Atoms gegen die $-NR_4R_5$-Gruppe

oder

B) durch Umsetzung der entsprechenden 1H-2-Phenylindolverbindung X nach Deprotonierung mit dem entsprechenden $\omega$-Bromalkylcarbonamid

$$Br\text{-}(CH_2)_{n-1}\text{-}\underset{\underset{O}{\|}}{C}NR^4R^5$$

erhalten; letztere Verbindungen sind durch Reaktion des entsprechenden $\omega$-Bromcarbonsäurechlorids

$$Br\text{-}(CH_2)_{n-1}\text{-}\underset{\underset{O}{\|}}{C}\text{-}Cl$$

mit einem Überschuß an Amin $HNR_4R_5$ leicht zugänglich.

**A1) Allgemeine Vorschrift zur N-Alkylierung**

1,44 g (0,06 mol) Natrium werden bei -70 °C in 200 ml flüssigem Ammoniak gelöst. Nach dem Verschwinden der Blaufärbung wird zu dieser Lösung eine Lösung, bestehend aus 0,035 mol entsprechender 1H-2-Phenylindolverbindung X (J.Med.Chem. 1984, 27, 1439) in 100 ml Tetrahydrofuran, gegeben. Nach 30 Minuten Rührzeit wird zum Reaktionsgemisch langsam eine Lösung von 0,042 mol $\alpha,\omega$-Dibromalkan $Br\text{-}(CH_2)_n\text{-}Br$ gelöst in 20 ml Tetrahydrofuran, getropft. Es wird 30 Minuten nachgerührt und das Kühlbad entfernt. Nach dem Verdampfen des Ammoniaks wird der verbleibende Rückstand mit Wasser versetzt und mit Ether extrahiert. Nach Separieren der Phasen wird die organische Lösung getrocknet, im Vakuum eingeengt und der verbleibende Rückstand aus Ethanol umkristallisiert oder durch Säulenchromatographie gereinigt.

Die so hergestellten N-Alkylverbindungen Ya bis Ye sind in der Tabelle 7 zusammengefaßt.

Tabelle 7

| N-Alkylverbindungen Ya bis Ye durch Alkylierung entsprechender 1H-2-Phenylindolverbindungen X | | | | | | |
|---|---|---|---|---|---|---|
| N-Alkylverbindung Y | | 1H-2-Phenylindol X | | | $Br(-CH_2)_nBr$ n | Schmp. [°C] |
| | | $R_3$ | $R_6$ | $R_7$ | | |
| **Ya** | 5-Methoxy-2-(4-methoxyphenyl)-3-methyl-1--(4-brombutyl)-indol | $CH_3$ | $OCH_3$ | $OCH_3$ | 4 | 59-60 |
| **Yb** | 5-Methoxy-2-(4-methoxyphenyl)-3-methyl-1--(6-bromhexyl)-indol | $CH_3$ | $OCH_3$ | $OCH_3$ | 6 | 60 |
| **Yc** | 5-Methoxy-2-(4-methoxyphenyl)-1-(6-bromhexyl)-indol | H | $OCH_3$ | $OCH_3$ | 6 | 82 |
| **Yd** | 5-Methoxy-2-(4-methoxyphenyl)-3-methyl-1--(8-bromoctyl)-indol | $CH_3$ | $OCH_3$ | $OCH_3$ | 8 | Öl |
| **Ye** | 2-(4-Methoxyphenyl)--3-methyl-1-(6-bromhexyl)-indol | $CH_3$ | H | $OCH_3$ | 6 | Öl |

**A2) Allgemeine Vorschriften zur Darstellung der Verbindungen der allgemeinen Formel IIa durch Austausch des ω-Bromatoms in der Alkylseitenkette**

**a) Umsetzung mit einem cyclischen Amin**

Eine Lösung von 3.0 mmol des entsprechenden ω-Bromalkylindols Y wird in 100-150 ml ml des cyclischen Amins 4 Stunden auf 120 °C erhitzt. Nach dem Abkühlen auf Raumtemperatur wird mit Dichlormethan und Wasser ausgeschüttelt, die vereinigten organischen Phasen über Magnesiumsulfat getrocknet und das Lösungsmittel abdestilliert. Die als braune Öle anfallenden Rohprodukte werden an Kieselgel mit Methanol bzw. Methanol/Triethylamin (1:1 bis 1:3) chromatographiert.

**b) Umsetzung mit Phtahlimid-Kalium/Hydrazin**

8,0 mmol der 1-(ω-Bromalkyl)-indolverbindung Y und 8,8 mmol Phthalimid/Kalium werden in 100 ml wasserfreiem Dimethylformamid 2 Stunden unter Rückfluß erhitzt. Nach dem Abkühlen wird mit Dichlormethan und Wasser ausgeschüttelt. Anschließend wird über Natriumsulfat getrocknet und das Lösungsmittel abdestilliert. Der Rückstand wird an Kieselgel mit Dichlormethan/Essigester (10:1) chromatographiert. Zur Freisetzung des Amins wird in 50 ml Ethanol (99%ig) aufgenommen und mit Hydrazin-Hydrat in 20 ml Ethanol versetzt. Es wird 2 Stunden unter Rückfluß erwärmt, nach dem Abkühlen mit 40 ml 2 n Salzsäure angesäuert (pH 2-3) und vom Niederschlag abgesaugt. Nach dem Abdestillieren des Lösungsmittels wird mit 40 ml 2 n Natronlauge alkalisch gestellt (pH 8-9) und dreimal mit jeweils 50 ml Essigester extrahiert. Anschließend wird über Natriumsulfat getrocknet und an Kieselgel mit Dichlorethan/Trimethylamin (5:1) chromatographiert.

c) **Umsetzung mit einem aliphatischen Amin**

7,0 mmol der 1-(ω-Bromalkyl)-indolverbindung Y werden in 30 ml Ethanol (99%ig) gelöst, mit 40 ml 40%iger wäßriger Lösung des aliphatischen Amins versetzt und 3 Stunden unter Rückfluß erhitzt. Nach dem Abkühlen wird das Ethanol weitgehend abgezogen, der Rückstand in 100 ml Dichlormethan aufgenommen und mit 100 ml Wasser extrahiert. Anschließend wird die organische Phase über Magnesiumsulfat getrocknet und das Lösungsmittel abdestilliert. Der Rückstand wird an Kieselgel mit Methanol chromatographiert.

Die nach den Vorschriften A2a), A2b) sowie A2c) hergestellten Verbindungen IIa gehen aus der Tabelle 8 hervor.

**Tabelle 8**

Verbindungen der allgemeinen Formel IIa aus den N-(ω-Bromalkyl)indolen Y

| Verbindung der allgemeinen Formel IIa | | N-(ω-Bromalkyl)-indol Y | Amin $HNR_4R_5$ | Vorschrift | Aussehen | Ausbeute [%] |
|---|---|---|---|---|---|---|
| IIa1 | 5-Methoxy-2-(4-methoxyphenyl)-3-methyl-1-[4-pyrrolidino-butyl]-indol | Ya | Pyrrolidin | A2a | farblose Kristalle Schmp. 59-60°C | 51 |
| IIa2 | 5-Methoxy-2-(4-methoxyphenyl)-3-methyl-1-[6-aminohexyl]-indol | Yb | Hydrazin | A2b | blaßgelbes Öl | |
| IIa3 | 5-Methoxy-2-(4-methoxyphenyl)-3-methyl-1-[6-methylaminohexyl]-indol | Yb | Methylamin | A2c | blaßgelbes Öl | 84 |
| IIa4 | 5-Methoxy-2-(4-methoxyphenyl)-3-methyl-1-[6-dimethylaminohexyl]-indol | Yb | Dimethylamin | A2c | blaßgelbes Öl | 41 |
| IIa5 | 5-Methoxy-2-(4-methoxyphenyl)-1-[6-pyrrolidino-hexyl]-indol | Yc | Pyrrolidin | A2a | blaßgelbes Öl | 77 |
| IIa6 | 5-Methoxy-2-(4-methoxyphenyl)-3-methyl-1-[6-pyrrolidinohexyl]-indol | Yb | Pyrrolidin | A2a | gelbes Öl | 90 |
| IIa7 | 5-Methoxy-2-(4-methoxyphenyl)-1-[6-piperidino-hexyl]-indol | Yc | Piperidin | A2a | blaßgelbes Öl | 90 |
| IIa8 | 5-Methoxy-2-(4-methoxyphenyl)-3-methyl-1-[6-piperidino-hexyl]-indol | Yb | Piperidin | A2a | blaßgelbes Öl | 89 |
| IIa9 | 5-Methoxy-2-(4-methoxyphenyl)-3-methyl-1-[8-pyrrolidino-octyl]-indol | Yd | Pyrrolidin | A2a | gelbes Öl | 65 |
| IIa13 | 2-(4-Methoxyphenyl)-3-methyl-1-[6-pyrrolidino-hexyl]-indol | Ye | Pyrrolidin | A2a | blaßgelbes Öl | 75 |

**B1) Allgemeine Vorschrift zur Herstellung der Verbindungen der allgemeinen Formel IIb durch Umsetzung der 1H-2-Phenylindole X mit ω-Bromalkylcarbonamiden $Br-(CH_2)_{n-1}-C(=O)NR_4R_5$ (IIb)**

Im Reaktionskolben werden 20,0 mmol NaH in Paraffin unter Stickstoff durch mehrmaliges Waschen mit Ether vom Paraffin befreit und mit 20 ml trockenem Dimethylacetamid versetzt. Zu dieser Mischung gibt man unter Eiskühlung tropfenweise eine Lösung von 14 mmol 1H-2-Phenylindol X in 80 ml trockenem Dimethylacetamid. Nach 30 minütigem Rühren wird unter Eiskühlung eine Lösung von 15 mmol ω-Bromalkylcarbonamid in 60 ml trockenem Dimethylacetamid zugetropft. Nach 2 Stunden Rühren bei Raumtemperatur wird der Überschuß an NaH vorsichtig mit Wasser zerstört. Die Reaktionsmischung wird mehrmals mit Ethylacetat extrahiert. Die vereinigten organischen Phasen werden mit Wasser gewaschen, über $Na_2SO_4$ getrocknet und vom Solvens befreit. Die Produkte werden durch Säulenchromatographie gereinigt.
Die nach obiger Vorschrift hergestellten Verbindungen IIb sind aus Tabelle 9 ersichtlich.

**Tabelle 9**

**Verbindungen der allgemeinen Formel IIb aus den 1H-2-Phenylindolen X**

| Verbindung der allgemeinen Formel IIb | 1H-2-Phenylindol X $R_3$ | $R_6$ | $R_7$ | ω-Bromalkylcarbonamid | Aussehen/Schmp. |
|---|---|---|---|---|---|
| IIb 16 5-Methoxy-2-(4-methoxyphenyl)-3-methyl-1-(6-pyrrolidinocarbonylpentyl)-indol | $CH_3$ | $OCH_3$ | $OCH_3$ | 6-Bromhexanoylpyrrolidin | gelbes Harz |
| IIb 17 5-Methoxy-2-(4-methoxyphenyl)-3-methyl-1-(6-piperidinocarbonylpentyl)-indol | $CH_3$ | $OCH_3$ | $OCH_3$ | 6-Bromhexanoylpiperidin | hellgelbes Harz |
| IIb 18 5-Methoxy-2-(4-methoxypentyl)-3-methyl-1-(6-morpholinocarbonylpentyl)-indol | $CH_3$ | $OCH_3$ | $OCH_3$ | 6-Bromhexanoylmorpholin | hellbraunes Harz |
| IIb 19 1-(6-Diethylcarbamoylpentyl)-5-methoxy-2-(4-methoxyphenyl)-3-methylindol | $CH_3$ | $OCH_3$ | $OCH_3$ | 6-Bromhexanoyldiethylamin | farbloses Harz |
| IIb 20 1-(6-Benzylcarbamoylpentyl)-5-methoxy-2-(4-methoxyphenyl)-3-methylindol | $CH_3$ | $OCH_3$ | $OCH_3$ | N-(6-Bromhexanoyl)-benzylamin | Schmp.(Ethanol) 127-129 |

## Beispiel 1 bis 20

**C1) Allgemeine Vorschrift zur Herstellung der Verbindungen der allgemeinen Formel Ia (d.h. Verbindungen der allgemeinen Formel I, die mindestens eine freie Hydroxygruppe aufweisen) und IIIb durch Etherspaltung von IIa bzw. IIb**

Unter Stickstoffatmosphäre und Eiskühlung löst man 4,0 mmol des Indolderivats IIa oder IIb in 100 ml wasserfreiem Dichlormethan, rührt ca. 10 Minuten nach und tropft 9,0 mmol (2.25 g, 0,85 ml) Bortribromid, gelöst in 3 ml absolutem Dichlormethan, zu. Nun rührt man 30 Minuten unter Eiskühlung und 2-3 Stunden bei Raumtemperatur. Unter erneuter Eiskühlung tropft man 20 ml Essigester hinzu und rührt 30 Minuten bei Raumtemperatur. Man schüttelt mindestens dreimal mit je 50 ml Essigester, 10%iger Natriumhydrogencarbonat-Lösung und Wasser aus. Die vereinigten organischen Phasen werden über Magnesiumsulfat getrocknet und der Rückstand in Ethanol gelöst und im Tiefkühlfach zur Kristallisation gebracht. Die erhaltenen farblosen bis gelb-braun gefärbten Produkte der allgemeinen Formel Ia bzw. IIIb werden abgesaugt, mehrmals mit Wasser gewaschen und im Exsikator über Phosphorpentoxid getrocknet.
Die so hergestellten Verbindungen der allgemeinen Formel Ia zeigt Tabelle 10 und diejenigen der allgemeinen Formel IIIb Tabelle 11.

**C2) Allgemeine Vorschrift zur Veresterung von Verbindungen der allgemeinen Formel Ia**

12.3 mmol der Verbindung der allgemeinen Formel Ia werden in 50 ml Methylenchlorid gelöst, mit 20 ml des entsprechenden Säureanhydrids oder Säurehalogenids und 10 ml Pyridin versetzt und über Nacht gerührt. Anschließend wird in 200 ml Wasser eingefällt, mit Methylenchlorid extrahiert, getrocknet und im Vakuum eingeengt.

**C3) Allgemeine Vorschrift zur Herstellung des Hydrochlorids einer Verbindung der allgemeinen Formel I**

Es wird zunächst eine Verbindung der allgemeinen Formel Ia - wie unter C2) beschrieben - verestert. Der nach dem Einengen des Methylenchloridextraktes verbleibende ölige Rückstand wird in 200 ml Ether gelöst; durch diese Lösung wird 30 Minuten Chlorwasserstoff-Gas geleitet. Der ausgefallene Niederschlag wird abgesaugt und getrocknet.

**Tabelle 10**

| Beispiel/ Verbindung | Verbindung der allgemeinen Formel I | Ausgangsprod. | Vorschrift | Aussehen | Schmp. [°C] | Ausbeute [%] |
|---|---|---|---|---|---|---|
| 1 | 5-Hydroxy-2-(4-hydroxyphenyl)-3-methyl-1-[4-pyrrolidino-butyl]-indol | IIa 1 | C1 | beige Kristalle | 148 (Zers.) | 35 |
| 2 | 5-Hydroxy-2-(4-hydroxyphenyl)-3-methyl-1-[6-amino-hexyl]-indol | IIa 2 | C1 | gelbe Kristalle | >139 (Zers.) | 76 |
| 3 | 5-Hydroxy-2-(4-hydroxyphenyl)-3-methyl-1-[6-methylamino-hexyl]-indol | IIa 3 | C1 | gelbe Kristalle | 194 (Zers.) | 24 |
| 4 | 5-Hydroxy-2-(4-hydroxyphenyl)-3-methyl-1-[6-dimethylamino-hexyl]-indol | IIa 4 *=oder | C1 | gelbe Kristalle | 189 (Zers.) | 73 |
| 5 | 5-Hydroxy-2-(4-hydroxyphenyl)-1-[6-pyrrolidino-hexyl]-indol | IIa 5 | C1 | gelbe Kristalle | 109-112 (Zers.) | 50 |
| 6 | 5-Hydroxy-2-(4-hydroxyphenyl)-3-methyl-1-[6-pyrrolidino-hexyl]-indol | IIa 6/ IIb 16 | C1 | beige Kristalle | 148-150 (Zers.) | 56 |

**Tabelle 10 (Fortsetzung)**

| Beispiel/ Verbindung | Verbindung der allgemeinen Formel I | Ausgangsprod. | Vorschrift | Aussehen | Schmp. [°C] | Ausbeute [%] |
|---|---|---|---|---|---|---|
| 7 | 5-Hydroxy-2-(4-hydroxyphenyl)-1-[6-piperidino-hexyl]-indol | IIa 7 | C1 | rotbraune Kristalle | 125-128 (Zers.) | 80 |
| 8 | 5-Hydroxy-2-(4-hydroxyphenyl)-3-methyl-1-[6-piperidino-hexyl]-indol | IIa 8/IIb 17 | C1 | gelbe Kristalle | 102 (Zers.) | 23 |
| 9 | 5-Hydroxy-2-(4-hydroxyphenyl)-3-methyl-1-[8-pyrrolidino-ocytl]-indol | IIa 9 | C1 | gelbe Kristalle | 166-169 (Zers.) | 42 |
| 10 | 5-Hydroxy-2-(4-hydroxyphenyl)-3-methyl-1-[6-morpholino-hexyl]-indol | IIb 18 | | | 107-110 (Zers.) | |
| 11 | 1-(6-Diethylaminohexyl)-5-hydroxy-2-hydroxyphenyl)-3-methyl-indol | IIb 19 | C1 | hellgelbes, amorphes Pulver | | |
| 12 | 1-(6-Benzylaminohexyl)-5-hydroxy-2-(4-hydroxyphenyl)-3-methyl-indol | IIb 20 | C1 | beiges, amorphes Pulver | | |

**Tabelle 10 (Fortsetzung)**

| Beispiel/ Verbindung | Verbindung der allgemeinen Formel I | Ausgangsprod. | Vorschrift | Aussehen | Schmp. [°C] | Ausbeute [%] |
|---|---|---|---|---|---|---|
| 13 | 2-(4-Hydroxyphenyl)-3-methyl-1-[6-pyrrolidino-hexyl]-indol | IIa 13 | C1 | gelbe Kristalle | - | 35 |
| 14 | 5-Acetoxy-2-(4-acetoxyphenyl)-3-methyl-1-[6-piperidino-hexyl]-indol | Verbindung 7 +Acetanhydrid | C2 | gelbes Öl | - | 53 |
| 15 | 5-Benzoyloxy-2-(4-benzoyloxyphenyl)-3-methyl-1-[6-piperidino-hexyl]-indol-Hydrochlorid | Verbindung 7 +Benzoylchlorid | C3 | blaßgelbes Öl | - | 55 |

**Tabelle 11**

| Beispiel/Verbindung | Verbindung der allgemeinen Formel IIIb | Ausgangsprodukt | Aussehen | Schmp. [°C] |
|---|---|---|---|---|
| 16 | 5-Hydroxy-2-(4-hydrdoxyphenyl)-3-methyl-1-(6-pyrrolidinocarbonylpentyl)-indol | IIb 16 | hellgrünes, amorphes Pulver | |
| 17 | 5-Hydroxy-2-(4-hydroxyphenyl)-3-methyl-1-(6-piperidinocarbonylpentyl)-indol | IIb 17 | orangenes, amorphes Pulver | 83-94 |
| 18 | 5-Hydroxy-2-(4-hydroxyphenyl)-3-methyl-1-(6-morpholinocarbonylpentyl)-indol | IIb 18 | hellgelbes, amorphes Pulver | |
| 19 | 1-(6-Diethylcarbamoylpentyl)-5-hydroxy-2-4-hydroxyphenyl)-3-methylindol | IIb 19 | gelbliches, amorphes Pulver | |
| 20 | 1-(6-Benzylcarbamoylpentyl)-5-hydroxy-2-4-hydroxyphenyl)-3-methylindol | IIb 20 | | 189-191 |

Die nachfolgenden Beispiele 21-23 zeigen galenische Formulierungen einiger erfindungsgemäßer Verbindungen:

**Beispiel 21**

21, g 5-Hydroxy-2-(4-hydroxyphenyl)-1-[6-pyrrolidino-hexyl]-indol und 89,0 g Lactose werden homogen gemischt und jeweils 210 mg dieser Mischung in Hartgelantine-Steckkapseln der Größe 3 gefüllt.

**Beispiel 22**

Tabletten können in üblicher Weise aus folgenden Bestandteilen hergestellt werden:

| | |
|---|---|
| 50 mg | 5-Hydroxy-2-(4-hydroxyphenyl)-3-methyl-1-[6-pyrrolidino-hexyl]-indol |
| 200 mg | Lactose |
| 200 mg | mikrokristalline Cellulose |
| 50 mg | Magnesiumstearat |
| 500 mg | Gesamtgewicht der Tablette |

**Beispiel 23**

Tabletten können in üblicher Weise aus folgenden Bestandteilen hergestellt werden:

| | |
|---|---|
| 10 mg | 5-Hydroxy-2-(4-hydroxyphenyl)-3-methyl-1-[8-pyrrolidino-octyl]-indol |
| 220 mg | Lactose |
| 220 mg | mikrokristalline Cellulose |
| 50 mg | Magnesiumstearat |
| 500 mg | Gesamtgewicht der Tablette |

**Patentansprüche**

**1.** Aminoalkylindole der allgemeinen Formel I

R3
3
4
5
R1
2
R2
6
N
1
7
(CH2)n-N
R4
R5
(I) ,

worin

$R_1$ ein Wasserstoffatom, eine Hydroxyl- oder eine Alkanoyloxygruppe mit 1-10 Kohlenstoffatomen, die gesättigt oder ungesättigt sein kann,

$R_2$ eine Hydroxyl- oder Alkanoyloxygruppe mit 1-10 Kohlenstoffatomen, die gesättigt oder ungesättigt sein kann,

$R_3$ ein Wasserstoffatom oder eine Methylgruppe,

$R_4$, $R_5$ Wasserstoff, Alkylgruppen mit 1-10 Kohlenstoffatomen, Aralkylgruppen mit 7-10 Kohlenstoffatomen oder Cycloalkylgruppen mit 3-7 Kohlenstoffatomen, wobei die Reste $R_4$ und $R_5$ gleich oder verschieden sind oder

$R_4$, $R_5$ unter Einschluß des N-Atoms einen 5- oder 6-gliedrigen heterocyclischen Ring bilden, der gegebenenfalls neben dem Stickstoff noch ein Sauerstoff- oder ein weiteres Stickstoffatom als Heteroatom enthält und

n = 4-15

bedeuten, und die Salze dieser Verbindungen mit Säuren.

**2.** Verbindungen der allgemeinen Formel I nach Anspruch 1:
5-Hydroxy-2-(4-hydroxyphenyl)-3-methyl-1-[4-pyrrolidino-butyl]-indol
5-Hydroxy-2-(4-hydroxyphenyl)-3-methyl-1-[6-amino-hexyl]-indol
5-Hydroxy-2-(4-hydroxyphenyl)-3-methyl-1-[6-methylamino-hexyl]-indol
5-Hydroxy-2-(4-hydroxyphenyl)-3-methyl-1-[6-dimethylamino-hexyl]-indol
5-Hydroxy-2-(4-hydroxyphenyl)-1-[6-pyrrolidino-hexyl]-indol
5-Hydroxy-2-(4-hydroxyphenyl)-3-methyl-1-[6-pyrrolidino-hexyl]-indol
5-Hydroxy-2-(4-hydroxyphenyl)-1-[6-piperidino-hexyl]-indol
5-Hydroxy-2-(4-hydroxyphenyl)-3-methyl-1-[6-piperidino-hexyl]-indol
5-Hydroxy-2-(4-hydroxyphenyl)-3-methyl-1-[8-pyrrolidino-octyl]-indol
5-Hydroxy-2-(4-hydroxyphenyl)-3-methyl-1-[6-morpholinohexyl)-indol
1-(6-Diethylaminohexyl)-5-hydroxy-2-(4-hydroxyphenyl)-3-methyl-indol
1-(6-Benzylaminohexyl)-5-hydroxy-2-(4-hydroxyphenyl)-3-methylindol
2-(4-Hydroxyphenyl)-3-methyl-1-[6-pyrrolidino-hexyl]-indol
5-Acetoxy-2-(4-acetoxyphenyl)-3-methyl-1-[6-piperidino-hexyl]-indol
5-Benzoyloxy-2-(4-benzoyloxy-phenyl)-3-methyl-1-[6-piperidino-hexyl]-indol-Hydrochlorid

**3.** Verfahren zur Herstellung von Aminoalkylindolen der allgemeinen Formel I nach Anspruch 1, dadurch gekennzeichnet,
daß man eine Verbindung der allgemeinen Formel IIa

(II a) ,

oder der allgemeinen Formel IIb

(II b),

worin

| | |
|---|---|
| $R_3$, $R_4$, $R_5$ und n | die in Formel I genannte Bedeutung haben, |
| $R_6$ | ein Wasserstoffatom oder eine Alkoxygruppe mit 1-4 Kohlenstoffatomen und |
| $R_7$ | eine Alkoxygruppe mit 1-4 Kohlenstoffatomen |

bedeuten,
unter den Bedingungen einer Etherspaltung zu einer Verbindung der Formel I a

(I a) ,

oder der Formel III b

(III b) ,

worin $R_8$ ein Wasserstoffatom oder eine Hydroxylgruppe bedeuten, umsetzt, die Carbonylfunktion der Verbindung der allgemeinen Formel III b vollständig reduziert und anschließend gegebenenfalls die freie(n) aromatische(n) Hydroxylgruppe(n) in eine Alkanoyloxygruppe mit 1-10 Kohlenstoffatomen und gegebenenfalls eine Verbindungen der allgemeinen Formel I mit einer organischen oder anorganischen Säure in das entsprechende Salz überführt.

4. 1-Carbamoylalkyl-2-phenylindole der allgemeinen Formel IIIb

(III b) ,

worin $R_3$, $R_4$, $R_5$ und n die in Formel I genannte Bedeutung haben und $R_8$ ein Wasserstoffatom oder eine Hydroxylgruppe bedeutet.

5. Verbindungen der allgemeinen Formel IIIb nach Anspruch 4:
5-Hydroxy-2-(4-hydroxyphenyl)-3-methyl-1-(6-pyrrolidino-carbonylpentyl)-indol
5-Hydroxy-2-(4-hydroxyphenyl)-3-methyl-1-(6-piperidino-carbonylpentyl)-indol
5-Hydroxy-2-(4-hydroxyphenyl-3-methyl-1-(6-morpholino-carbonylpentyl)-indol
1-(6-Diethylcarbamoylpentyl)-5-hydroxy-2-(4-hydroxyphenyl)-3-methylindol
1-(6-Benzylcarbamoylpentyl)-5-hydroxy-2-(4-hydroxyphenyl)-3-methylindol.

6. Pharmazeutische Präparate, gekennzeichnet dadurch, daß sie mindestens eine der Verbindungen gemäß Anspruch 1, 2, 4 oder 5 sowie einen inerten, pharmazeutisch verträglichen Träger enthalten.

7. Verwendung der Verbindungen gemäß Anspruch 1, 2, 4, oder 5 zur Herstellung von Arzneimitteln zur Behandlung von östrogenabhängigen Krankheiten und Tumoren.

## Claims

1. Aminoalkylindoles of general formula I

(I) ,

in which

$R_1$ means a hydrogen atom, a hydroxyl or alkanoyloxy group with 1-10 carbon atoms, which can be saturated or unsaturated,

$R_2$ means a hydroxyl or alkanoyloxy group with 1-10 carbon atoms, which can be saturated or unsaturated,

$R_3$ means a hydrogen atom or a methyl group,

$R_4$, $R_5$ means hydrogen, alkyl groups with 1-10 carbon atoms, aralkyl groups with 7-10 carbon atoms or cycloalkyl groups with 3-7 carbon atoms, and the radicals $R_4$ and $R_5$ are the same or different, or

$R_4$, $R_5$ with inclusion of the N atom form a 5- or 6-membered heterocyclic ring, which, optionally besides the nitrogen atom, also contains an oxygen atom or another nitrogen atom as heteroatom, and

$n = 4\text{-}15$,

and the salts of these compounds with acids.

2. Compounds of general formula I according to claim 1:
5-hydroxy-2-(4-hydroxyphenyl)-3-methyl-1-[4-pyrrolidino-butyl]-indole
5-hydroxy-2-(4-hydroxyphenyl)-3-methyl-1-[6-amino-hexyl)-indole
5-hydroxy-2-(4-hydroxypnenyl)-3-methyl-1-[6-methylamino-hexyl]-indole
5-hydroxy-2-(4-hydroxyphenyl)-3-methyl-[6-dimethylamino-hexyl]-indole
5-hydroxy-2-(4-hydroxyphenyl)-1-[6-pyrrolidino-hexyl]-indole
5-hydroxy-2-(4-hydroxyphenyl)-3-methyl-1-[6-pyrrolidino-hexyl]-indole
5-hydroxy-2-(4-hydroxyphenyl)-1-[6-piperidino-hexyl]-indole
5-hydroxy-2-(4-hydroxyphenyl)-3-methyl-1-[6-piperidino-hexyl]-indole
5-hydroxy-2-(4-hydroxyphenyl)-3-methyl-1-[8-pyrrolidino-octyl]-indole
5-hydroxy-2-(4-hydroxyphenyl)-3-methyl-1-[6-morpholinohexyl]-indole
1-(6-diethylaminohexyl)-5-hydroxy-2-(4-hydroxyphenyl)-3-methyl-indole
1-(6-benzylaminohexyl)-5-hydroxy-2-(4-hydroxyphenyl)-3-methylindole
2-(4-hydroxyphenyl)-3-methyl-1-[6-pyrrolidino-hexyl]-indole
5-acetoxy-2-(4-acetoxyphenyl)-3-methyl-1-[6-piperidino-hexyl]-indole
5-benzoyloxy-2-(4-benzoyloxy-phenyl)-3-methyl-1-[6-piperidino-hexyl]-indole hydrochloride

3. Processes for the production of aminoalkylindoles of general formula I according to claim 1, characterized in that a compound of general formula IIa

(II a) ,

or of general formula IIb

(II b),

in which

$R_3$, $R_4$, $R_5$ and n have the meaning mentioned in formula I,

$R_6$ means a hydrogen atom or an alkoxy group with 1-4 carbon atoms and

$R_7$ means an alkoxy group with 1-4 carbon atoms,

under the conditions of an ether cleavage is reacted to a compound of formula Ia

(I a) ,

or of formula IIIb

(III b) ,

in which $R_8$ means a hydrogen atom or a hydroxyl group, the carbonyl function of the compound of general formula IIIb is completely reduced and then optionally the free aromatic hydroxyl group(s) is/are converted into an alkanoyloxy group with 1-10 carbon atoms and optionally a compound of

general formula I is converted with an organic or inorganic acid into the corresponding salt.

**4.** 1-Carbamoylalkyl-2-phenylindoles of general formula IIIb

(III b) ,

in which $R_3$, $R_4$, $R_5$ and n have the meaning indicated in formula I and $R_8$ means a hydrogen atom or a hydroxyl group.

**5.** Compounds of general formula IIIb according to claim 4:
5-hydroxy-2-(4-hydroxyphenyl)-3-methyl-1-(6-pyrrolidino-carbonylpentyl)-indole
5-hydroxy-2-(4-hydroxyphenyl)-3-methyl-1-(6-piperidino-carbonylpentyl)-indole
5-hydroxy-2-(4-hydroxyphenyl)-3-methyl-1-(6-morpholino-carbonylpentyl)-indole
1-(6-diethylcarbamoylpentyl)-5-hydroxy-2-(4-hydroxyohenyl)-3-methylindole
1-(6-benzylcarbamoylpentyl)-5-hydroxy-2-(4-hydroxyphenyl)-3-methylindole

**6.** Pharmaceutical preparations, characterized in that they contain at least one of the compounds according to claim 1, 2, 4 or 5 as well as an inert, pharmaceutically compatible vehicle.

**7.** Use of the compounds according to claim 1, 2, 4 or 5 for the production of pharmaceutical agents for treatment of estrogen-dependent diseases and tumors.

**Revendications**

**1.** Aminoalkylindoles de formule générale I

(I)

où

- $R_1$ représente un atome d'hydrogène, un groupe hydroxyle ou alcanoyloxy avec 1 à 10 atomes de carbone qui peut être sauré ou insaturé,
- $R_2$ représente un groupe hydroxyle ou alcanoyloxy avec 1 à 10 atomes de carbone qui peut être saturé ou insaturé,
- $R_3$ représente un atome d'hydrogène ou un groupe méthyle,
- $R_4$, $R_5$ représentent l'hydrogène, des groupes alxyle avec 1 à 10 atomes de carbone, des groupes aralkyle avec 7 à 10 atomes de carbone ou des groupes cycloalkyle avec 3 à 7 atomes de carbone,
  les radicaux $R_4$ et $R_5$ étant identiques ou différents ou
- $R_4$, $R_5$ en incluant l'atome de N, forment un cycle hétérocyclique à 5 ou 6 chaînons qui contient éventuellement, outre l'atome d'azote, encore un atome d'oxygène ou un autre atome

d'azote en tant qu'hétéroatome,

n = 4 à 15,

et les sels de ces composés avec des acides.

**2.** Composés de formule générale I selon la revendication 1 :

5-hydroxy-2-(4-hydroxyphényl)-3-méthyl-1-[4-pyrrolidino-butyl]-indole
5-hydroxy-2-(4-hydroxyphényl)-3-méthyl-1-[6-amino-hexyl]-indole
5-hydroxy-2-(4-hydroxyphényl)-3-méthyl-1-[6-méthylamino-hexyl]-indole
5-hydroxy-2-(4-hydroxyphényl)-3-méthyl-1-[6-diméthylamino-hexyl]-indole
5-hydroxy-2-(4-hydroxyphényl)-1-[6-pyrrolidino-hexyl]-indole
5-hydroxy-2-(4-hydroxyphényl)-3-méthyl-1-[6-pyrrolidino-hexyl]-indole
5-hydroxy-2-(4-hydroxyphényl)-1-[6-pipéridino-hexyl]-indole
5-hydroxy-2-(4-hydroxyphényl)-3-méthyl-1-[6-pipéridino-hexyl]-indole
5-hydroxy-2-(4-hydroxyphényl)-3-méthyl-1-[8-pyrrolidino-octyl]-indole
5-hydroxy-2-(4-hydroxyphényl)-3-méthyl-1-[6-morpholinohexyl]-indole
1-(6-diéthylaminohexyl)-5-hydroxy-2-(4-hydroxyphényl)-3-méthyl-indole
1-(6-benzylaminohexyl)-5-hydroxy-2-(4-hydroxyphényl)-3-méthylindole
2-(4-hydroxyphényl)-3-méthyl-1-[6-pyrrolidino-hexyl]-indole
5-acétoxy-2-(4-acétoxyphényl)-3-méthyl-1-[6-pipéridino-hexyl]-indole
chlorhydrate de 5-benzoyloxy-2-(4-benzoyloxy-phényl)-3-méthyl-1-[6-pipéridino-hexyl]-indole

**3.** Procédé pour la préparation d'aminoalkylindoles de formule générale I selon la revendication 1, caractérisé en ce que

on fait réagir un composé de formule générale IIa

(IIa)

ou de formule générale IIb

(IIb)

où

| | |
|---|---|
| $R_3$, $R_4$, $R_5$ et n | ont la signification donnée dans la formule I |
| $R_6$ | représente un atome d'hydrogène ou un groupe alcoxy avec 1 à 4 atomes de carbone et |
| $R_7$ | représente un groupe alcoxy avec 1 à 4 atomes de carbone, |

dans des conditions du clivage du groupe éther, pour obtenir un composé de formule Ia

(Ia)

ou de formule IIIb

(IIIb)

où $R_8$ représente un atome d'hydrogène ou un groupe hydroxyle, on réduit complètement la fonction carbonyle du composé de formule générale IIIb et ensuite, on transforme éventuellement le(s) groupe-(s) hydroxyle aromatique(s) libre(s) en un groupe alcanoyloxy avec 1 à 10 atomes de carbone et éventuellement un composé de formule générale I, par un acide organique ou minérale, en le sel correspondant.

**4.** 1-Carbamoylalkyl-2-phénylindole de formule générale IIIb

(IIIb)

où $R_3$, $R_4$, $R_5$ et n ont la signification donnée dans la formule I et $R_8$ représente un atome d'hydrogène ou un groupe hydroxyle.

**5.** Composés de formule générale IIIb selon la revendication 4 :
5-hydroxy-2-(4-hydroxyphényl)-3-méthyl-1-[6-pyrrolidino-carbonylpentyl]-indole
5-hydroxy-2-(4-hydroxyphényl)-3-méthyl-1-[6-pipéridino-carbonylpentyl]-indole
5-hydroxy-2-(4-hydroxyphényl)-3-méthyl-1-[6-morpholino-carbonylpentyl]-indole
1-(6-diéthylcarbamoylpentyl)-5-hydroxy-2-(4-hydroxyphényl)-3-méthylindole
1-(6-benzylcarbamoylpentyl)-5-hydroxy-2-(4-hydroxyphényl)-3-méthylindole.

**6.** Préparations pharmaceutiques caractérisées en ce qu'elles contiennent au moins un des composés selon la revendication 1, 2, 4 ou 5 ainsi qu'un véhicule inerte, pharmaceutiquement compatible.

**7.** Utilisation des composés selon la revendication 1, 2, 4 ou 5 pour la préparation de médicaments pour le traitement des maladies oestrogéno-dépendantes et des tumeurs.